Europäisches Patentamt

(19) European Patent Office

Office européen des brevets

(11) Publication number: 0 370 656
A1

## (12) EUROPEAN PATENT APPLICATION

(21) Application number: 89311507.1

(22) Date of filing: 07.11.89

(51) Int. Cl.5: **A61K 37/02**, //(A61K37/02, 31:52)

(30) Priority: 09.11.88 US 269276

(43) Date of publication of application: 30.05.90 Bulletin 90/22

(84) Designated Contracting States: ES GR

(71) Applicant: SCHERING CORPORATION
2000 Galloping Hill Road
Kenilworth New Jersey 07033(US)

(72) Inventor: Bonnem, Eric M.
622 Belvidere Avenue
Plainfield New Jersey 07062(US)

(74) Representative: Ritter, Stephen David et al
Mathys & Squire 10 Fleet Street
London EC4Y 1AY(GB)

(54) **Treatment of myelosuppression associated with acquired immune deficiency.**

(57) Pharmaceutical compositions and methods of treatment are useful for treating myelosupression and viral infections associated with AIDS, particularly cytomegalovirus retinitis. Such compositions and treatments utilize an antiviral effective amount of 9-(1,3-dihydroxy-2-propoxymethyl) guanine (DHPG) in combination with an anti-myelosuppressive effective amount of granulocyte macrophage colony stimulating factor (GM-CSF). The use of DHPG and of GM-CSF for the preparation of a pharmaceutical composition for use in this combination treatment as well as kits containing pharmaceutical compositions containing DHPG and GM-CSF are also disclosed.

# TREATMENT OF MYELOSUPPRESSION ASSOCIATED WITH ACQUIRED IMMUNE DEFICIENCY

This invention relates to pharmaceutical compositions comprising an antiviral effective amount of DHPG, i.e. 9-(1,3-dihydroxy-2-propoxymethyl) guanine, in combination with an anti-myelosuppressive effective amount of GM-CSF, i.e. granulocyte macrophage colony stimulating factor, for use in treating patients suffering from myelosuppression and a viral infection associated with AIDS, i.e. Acquired Immune Deficiency Syndrome.

Acquired Immune Deficiency Syndrome (AIDS) is a generally lethal disease. The etiologic agent is believed to be viral, specifically retroviruses of the HTLV/LAV-type now known as the human immunodeficiency virus (HIV). The social, economic, ethical and scientific perspective relating to AIDS is extensively reviewed in Science, 239 537-622 (1988).

The major effect of the AIDS virus is the incapacitation of the immune system, particularly T4 helper cells, which is the very system designed to fight against such invaders. Usually, the fatal result of AIDS infection is the host defense defect that renders the body highly susceptible to opportunistic infections and neoplasms. Besides halting the AIDS virus itself, methods of treatment can be directed to lessening the effect of such opportunistic infections and neoplasms. Cytomegalovirus (CMV) infection eventually develops in many patients having severe HIV disease as described in Quinnan et al., JAMA 1984, 252: 72-7; and Macher et al., N. Engl. J. Med. 1983, 309: 1454. In fact, CMV is the most frequent cause of HIV-associated retinitis. See Glatt et al., N. Engl. J. Med. 1988, 318: 1439-48; and Palestine et al., Opthalmology 1984, 91: 1092-9.

Clinical reports suggest that an effective treatment for cytomegalovirus retinitis is intravenous administration of 9-(1,3-dihydroxy-2-propoxymethyl) guanine (ganciclovir or DHPG from Syntex, Palo Alto, California) of 7.5 to 15 mg per kilogram per day. Such treatment is described in Shepp et al., Am. Intern. Med. 1985, 103: 368-73; Fletcher et al., Clin. Pharmacol. Ther. 1986, 40: 281-6; Bach et al., An. Intern. Med. 1985, 103: 381-2; Reed et al., An. Intern. Med., 1986, 105: 214-5; Rosecan et al., Am. J. Opthalmol. 1986, 101: 405-18; Palestine et al., Am. J. Opthalmol. 1986, 101: 95-101; Felsenstein et al., An. Intern. Med. 1985, 103: 377-80; Masur et al., An. Intern. Med. 1986, 104; 41-4; Collaborative DHPG Treatment Study Group, N. Engl. J. Med. 1986, 314: 801-5. The same clinical studies document that a major adverse effect of DHPG therapy is a dose related myelosuppression, especially neutropenia.

Granulocyte macrophage colony stimulating factor (GM-CSF) is a lymphokine that exhibits a broad spectrum of immune cell stimulation as described in Burgess and Metcalf, Blood 1980, 56: 947-58. GM-CSF has been shown to increase the leukocyte count in patients with the acquired immunodeficiency syndrome (Brandt et al., N. Engl. J. Med. 1988, 318: 869-76) and people suffering from chemotherapy-induced myelosuppression (Antman et al., New Eng. J. Med. 1988, 319(10): 593- 598), and it has been suggested that various colony stimulating factors alone may be a useful treatment of patients suffering from AIDS-type disease or together with erythropoietin and/or an antiviral agent and/or interleukin-2(IL-2)(PCT Application. No. 87/03204).

The present invention may be summarized in the following manner. It has now been found that administration of the combination of ganciclovir (DHPG) and GM-CSF is a highly advantageous method of arresting the viral infection associated with cytomegalovirus retinitis while advantageously alleviating DHPG-related myelosuppression. Thus, one aspect of the present invention is a pharmaceutical composition comprising an antiviral effective amount of DHPG in combination with a anti-myelosuppressive effective amount of GM-CSF. Another aspect involves administering to a patient suffering from cytomegalovirus retinitis an antiviral effective amount of DHPG in combination with an anti-myelosuppressive effective amount of GM-CSF.

The present invention also contemplates the use of DHPG for the preparation of a pharmaceutical composition for use in a combined therapy for treating a patient suffering from myelosuppression and a viral infection by administering DHPG in association with GM-CSF.

The present invention further contemplates the use of GM-CSF for the preparation of a pharmaceutical composition for use in a combined therapy for treating a patient suffering from myelosuppression and a viral infection by administering GM-CSF in association with DHPG.

The present invention still further contemplates the use of DHPG in association with GM-CSF for the preparation of a pharmaceutical composition for treating a patient suffering from myelosuppression in association with a viral infection, said pharmaceutical composition comprising DHPG and GM-CSF together with pharmaceutical carriers therefor.

The present invention also provides the use of DHPG in combination with GM-CSF for treating a patient suffering fro myelosuppression and a viral infection. In addition, the present invention provides a process for the preparation of a pharmaceutical composition which comprises admixing DHPG and GM-CSF with pharmaceutically acceptable carriers therefor.

Finally, the present invention provides a kit for use in treating patients suffering from myelosuppression and a viral infection, said kit comprising a pharmaceutical package having a first container and a second container, wherein the first container comprises an antiviral effective amount of a pharmaceutical composition containing DHPG and the second container comprises an anti-myelosuppressive amount of GM-CSF pharmaceutical composition.

There is now provided a detained description of the present invention which involves combining an antiviral effective amount of DHPG with an anti-myelosuppressive effective amount of GM-CSF.

Ganciclovir or DHPG [9-(1,3-Dihydroxy-2-propoxymethyl)guanine], which is produced by Syntex Corporation, Palo Alto, California, and which may be obtained as described in U.S. Patent 4,355,032, can be administered in an amount so as to lessen the symptoms associated with cytomegalovirus retinitis. Such symptoms include production of a characteristic retinal lesion consisting of hemorrhagic exudates in a vascular pattern of distribution manifested clinically by decreased vision and/or blindness. Preferably, DHPG is administered in an amount of from about 5.0 to about 20 mg per kilogram per day. The preferred administration regime is twice daily by intravenous infusion. Most preferably, the DHPG is administered in an amount of from about 7.5 to about 15 mg per kilograms per day. The amount is adjusted based upon patient tolerance manifested by decreased white blood cell count.

Any suitable GM-CSF may be employed in the present invention. Complementary DNAs (CDNAs) for GM-CSF have recently been cloned and sequenced by a number of laboratories, e.g. Gough et al., Nature 309:763 (1984) (mouse); Lee et al., Proc. Natl. Acad. Sci, 82:4360 (1985) (human); Wong et al., Science 228:810 (1985) (human and gibbon); Cantrell et al., Proc. Natl. Acad. Sci, 82:6250 (1985) (human). Moreover, non-recombinant GM-CSF has been purified from various culture supernatants, e.g. U.S. Patent 4,438,032 (Mo cell line); Burgers et al., Exp. Hematol. 9:893 (1981) (mouse); Sparrow et al., Proc. Natl. Acad. Sci., 82:292 (1985) (purification and partial amino acid sequence for mouse); Wu et al., Exp. Hematol., 12:267 (1984) (rat); Gasson et al., Science 226:1339 (1984) (human); Sieff et al., science 230:1171 (1985) (human); Burgess et al., Blood, 69:43 (1987) (human). Among the human GM-CSFs, nucleotide sequence and amino acid sequence heterogeneity have been observed. For example, at the amino acid level of GM-CSF both threonine and isoleucine have been observed at position 100 with respect to the N-terminal alanine, suggesting that allelic forms, or polymorphs, of GM-CSF may exist within human populations. Also, various leader sequences occur at the N-terminal portion of the amino acid sequence. These leaders may be of various lengths and amino acid composition, which may or may not affect biological activity. All of the above discussed references are incorporated herein by reference for their disclosures of representative GM-CSFs for use in the present invention. Preferably, the GM-CSF used in the present invention will be a human GM-CSF, most preferably the GM-CSF described in Proc. Natl. Acad. Sci. USA 82:4360 (1985), which is hereby incorporated by reference.

According to this invention, GM-CSF is administered to a patient showing symptoms of CMV retinitis and who is being treated with DHPG as described above and who is experiencing a myelosuppression. Myleosuppression for purposes of this invention is meant to include a reduction in the white blood cell count below about 2000 per $cm^3$ or a granulocyte count less than about 1000 per $cm^3$.

The dosage for GM-CSF will vary according to the response observed. Generally, it is desirable to administer GM-CSF in a sufficient amount to maintain white blood cell counts from about 2000 to about 10,000 per $cm^3$, preferably from about 2000 to about 5,000 per $cm^3$. A typical dosage of GM-CSF is about 3 to about 15 $\mu$g per kilogram per day intravenously or subcutaneously. Such white blood cell levels will permit the full administration of DHPG and the prevention of further visual deterioration.

The usual time to efficacious rises in white blood cell count is 5 to 10 days after initiation of GM-CSF therapy. If no rise in white blood cell count has occurred, dosage may be adjusted by increasing increments of 25% every four days after day 10. If white blood cell count is greater than about 20,000 per $cm^3$, dosage should be decreased by 25%. Dosage should be adjusted so that white blood cell count does not exceed about 20,000 per $cm^3$.

The administration of the pharmaceutical compositions of this invention is via the subcutaneous or intravenous route. DHPG and GM-CSF may be given together or sequentially, whichever is deemed more appropriate by the attending clinician. The solutions to be administered may be reconstituted lypholized powders, particularly for GM-CSF and they may additionally contain preservatives, buffers, dispersants etc. Preferably, GM-CSF is reconstituted with preservative-free sterile water with the maximum concentration not to exceed 1500 micrograms per ml. and administered subcutaneously or as a continuous infusion, depending on the urgency of the situation. For continuous infusion, the daily dose is to be added to 50 ml of normal saline and the solution infused by mechanical pump.

The present invention also provides kits for use in treating patients suffering from myelosuppression and a viral infection as discussed above. The kits comprise a pharmaceutical package having a first container comprising a pharmaceutical composition containing an antiviral effective amount of DHPG and a second container comprising a pharmaceutical composition containing an anti-myelosuppressive amount of GM-CSF. The kits or packages may also contain instructions for use of the pharmaceutical composition of DHPG and of GM-CSF in accordance with the present invention.

The following examples are intended to illustrate the present invention and are not to be interpreted as limiting the invention in any way.

EXAMPLE 1

A 30 year old male was diagnosed as having AIDS and CMV retinitis. At Day 0, treatment with DHPG was commenced and life threatening myelosuppression was the result. A 100% dose of DHPG refers to a full protective dose or the recommended dose of DHPG of 15 mg per kilogram per day. The dosage of DHPG was maintained until changed as indicated in Table 1 below. As the accompanying table illustrates, administration of 10 mg of purified recombinant GM-CSF as described in Proc. Natl. Acad. Sci USA 82:4360 (1985) per kg by intravenous infusion drastically improved the white blood cell (WBC) count and permitted a return to a full protective dose of DHPG.

TABLE 1

| Day | WBC (per cm$^3$) | % Dose of DHPG Changed to | GM-CSF (μg/kg) |
|-----|------------------|---------------------------|----------------|
| 0   | 1000 | 50  | 0  |
| 29  | 700  | 50  | 0  |
| 34  | 300  | 0   | 0  |
| 49  | 1900 | 100 | 0  |
| 52  | 700  | 50  | 0  |
| 76  | 4300 | 50  | 10 |
| 80  | 9100 | 100 | 10 |

EXAMPLE 2

A 30 year old male was diagnosed as having AIDS and CMV retinitis. At Day 0, treatment with DHPG was commenced and a myelosuppression was the result. As the accompanying table illustrates, administration of GM-CSF, as described in Example 1, improved the white blood cell (WBC) count and permitted a return to a full protective dose of DHPG as described in Example 1.

TABLE 2

| Day | WBC (per cm$^3$) | % Dose of DHPG Changed to | GM-CSF (μg/kg) |
|-----|-----|-----|-----|
| 0 | 400 | 100 | 0 |
| 7 | 800 | 50 | 0 |
| 12 | -- | -- | 10 |
| 18 | 1400 | 100/66 | 3 |
| 20 | -- | -- | 0 |
| 42 | 600 | 50 | 10 |
| 44 + | 2500 | 100 | 10 |

EXAMPLE 3

A 56 year old male was diagnosed as having AIDS and CMV retinitis. At Day 0, treatment with DHPG was commenced and a myelosuppression was the result. As the accompanying table illustrates, administration of GM-CSF, as described in Example 1, improved the white blood cell (WBC) count and permitted a return to a full protective dose of DHPG as described in Example 1. Unfortunately, the patient developed thrombocytopenia during GM-CSF treatment and expired.

TABLE 3

| Day | WBC (per cm$^3$) | % Dose of DHPG Changed to | GM-CSF (μg/kg) |
|-----|-----|-----|-----|
| 0 | 2700 | 50 | 10 |
| 2 | 4000 | 50 | 10 |
| 18 | 7600 | 100 | 10 |
| 40 + | expired | | |

**Claims**

1. A pharmaceutical composition comprising an antiviral effective amount of DHPG in combination with an anti-myelosuppressive effective amount of GM-CSF.

2. The pharmaceutical composition as claimed in claim 1 wherein the antiviral effective amount of DHPG is an amount sufficient to lessen the symptoms associated with cytomegalovirus retinitis.

3. The pharmaceutical composition as claimed in claims 1 or 2 wherein the anti-myelosuppressive effective amount of GM-CSF is an amount sufficient to raise the white blood count to at least about 2000 per cm$^3$.

4. The pharmaceutical composition as claimed in anyone of claims 1 to 3 wherein the anti-myelosuppressive effective amount of GM-CSF is an amount sufficient to raise the white blood cell count to from about 2000 to about 10,000 per cm$^3$.

5. The pharmaceutical composition as claimed in anyone of claims 1 to 4 wherein the anti-myelosuppressive effective amount of GM-CSF is from about 3 to about 15 μg per Kg per day.

6. The pharmaceutical composition as claimed in anyone of claims 1 to 5 further comprising an appropriate solution for intraveneous infusion.

7. A method of treating a patient suffering from myelosuppression and a viral infection associated with AIDS comprising administering to such a patient an antiviral effective amount of DHPG and an anti-myelosuppression effective amount of GM-CSF.

8. A method of treating a patient as claimed in claim 7 wherein a pharmaceutical composition of anyone of claims 1 to 6 is administered to the patient.

9. The method of claim 7 wherein administration of the DHPG is intravenous and GM-CSF is subcutaneous.

10. The method of claim 7 wherein administration of the DHPG is intravenous and GM-CSF is intraveneous.

11. A method of treating a patient suffering from myelosuppression and cytomegalovirus retinitis associated with AIDS comprising administering to such a patient an antiviral effective amount of DHPG and an anti-suppressive effective amount of GM-CSF.

12. The use of DHPG for the preparation of a pharmaceutical composition for use in a combined therapy for treating a patient suffering from myelosuppression and a viral infection by administering DHPG in association with GM-CSF.

13. The use of GM-CSF for the preparation of a pharmaceutical composition for use in a combined therapy for treating a patient suffering from myelosuppression and a viral infection by administering GM-CSF in association with DHPG.

14. The use of DHPG in association with GM-CSF for the preparation of a pharmaceutical composition for treating a patient suffering from myelosuppression in association with a viral infection, said pharmaceutical composition comprising DHPG and GM-CSF together with pharmaceutically acceptable carriers therefor.

15. The use of DHPG in combination GM-CSF for treating a patient suffering from myelosuppression and a viral infection.

16. A process for the preparation of a pharmaceutical composition as claimed in anyone of claims 1 to 6 with comprises admixing DHPG and GM-CSF with pharmaceutically acceptable carriers therefor.

17. A kit for use in treating patients suffering from myelosuppression and a viral infection, said kit comprising a pharmaceutical package having a first container and a second container, wherein the first container comprises a pharmaceutical composition containing an antiviral effective amount of DHPG and the second container comprises a pharmaceutical composition containing anti-myelosuppressive amount of GM-CSF.

European Patent Office

**PARTIAL EUROPEAN SEARCH REPORT**
which under Rule 45 of the European Patent Convention
shall be considered, for the purposes of subsequent
proceedings, as the European search report

Application number

EP 89 31 1507

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | WO-A-87 03 204 (GENETICS INSTITUTE, INC.)<br><br>* Page 9, claim 11; page 4, lines 7-15 * | 1-6, 12-17 | A 61 K 37/02//<br>(A 61 K 37/02,<br>A 61 K 31:52) |

TECHNICAL FIELDS
SEARCHED (Int. Cl.4)

A 61 K

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:  1-6,12-17
Claims searched incompletely:
Claims not searched:  7-11
Reason for the limitation of the search:

Method for treatment of the human
or animal body by surgery or therapy
(See art. 52(4) of the European
Patent Convention).

| Place of search | Date of completion of the search | Examiner |
|---|---|---|
| The Hague | 02-02-1990 | BRINKMANN |